# EUROPÄISCHE PATENTSCHRIFT

(11) **EP 1 049 468 B1**
(45) Veröffentlichungstag und Bekanntmachung des Hinweises auf die Patenterteilung: **25.04.2007**
(21) Anmeldenummer: 99902528.1
(22) Anmeldetag: 11.01.1999
(51) Int. Cl.: A61K 31/44, A61K 47/36, A61K 47/26, A61P 17/02

(54) **VERWENDUNG VON HYDROXYPYRIDON-HALTIGEN PUDERZUBEREITUNGEN ZUR BEHANDLUNG VON UNTERSCHENKEL- UND DRUCKGESCHWÜREN**
UTILIZATION OF POWDER PREPARATIONS CONTAINING HYDROXYPYRIDONES FOR TREATING LEG ULCERS AND DECUBITUS ULCERS
UTILISATION DE PREPARATIONS PULVERULENTES CONTENANT DE L'HYDROXYPYRIDONE POUR LE TRAITEMENT D'ULCERES CRURAUX ET D'ULCERES DE COMPRESSION

(30) Priorität: 24.01.1998 DE 19802708
(43) Veröffentlichungstag der Anmeldung: 08.11.2000
(73) Patentinhaber: Sanofi-Aventis Deutschland GmbH, 65929 Frankfurt am Main (DE)
(72) Erfinder: BOHN, Manfred, D-65719 Hofheim (DE); KRAEMER, Karl, Theodor, D-63225 Langen (DE)
(86) Internationale Anmeldenummer: PCT/EP1999/000105
(87) Internationale Veröffentlichungsnummer: WO 1999/037300

(56) Entgegenhaltungen:
- WO-A-97/20560
- US-A- 4 797 409
- US-A- 5 494 658

## Beschreibung

Die vorliegende Erfindung betrifft die Verwendung von einer Puderzubereitung, enthaltend Hydroxypyridone zur Behandlung von Unterschenkelgeschwüren (Ulcus cruris) und Druckgeschwüren (Dekubitalulzera).

Die Diagnose von Unterschenkelgeschwüren (Ulcus cruris) ist ein Sammelbegriff, hinter dem sich Ulzera verschiedener Genese verbergen (Phlebologie (1996); 25: 254-258).
In Deutschland sind etwa 1,6 Millionen Menschen von schlecht heilenden Unterschenkelgeschwüren betroffen. Voraussetzung einer effektiven Wundbehandlung des Ulcus cruris ist es, die möglichen Ursachen für die Entstehung des Defektes zu erkennen. Global kann man sagen, daß eine gestörte Durchblutung der Gefäße des Unterschenkel vorliegt. Die daraus resultierende Unterversorgung des Gewebes mit Sauerstoff hat Läsionen, Gewebsverlust und schließlich Nekrotisierung und Ausweitung des Defektes zur Folge. Infektionen mit Bakterien und Pilzen besiedeln die betroffenen Hautareale und tragen neben der Vergrößerung der Wunde auch zur Bildung eines übelriechenden Sekretes bei.

Die Therapie des Ulcus cruris besteht aus einer Doppelstrategie aus lokaler Wundbehandlung sowie aus der Beseitigung der Ursachen der Ulzeration. Zu dieser kann die Kompressionstherapie bei einem durch chronische venöse Hypertonie bedingten Ulkus ebenso wie die Sanierung der arteriellen Strombahn bei einem arteriellen Ulcus cruris gehören.

Grundsätzlich ist bei der Lokaltherapie eines Ulkus stets zu beachten, daß eine Polypragmasie in der Anwendung der Mittel möglichst vermieden wird da Ulkus Patienten oft multivalente Kontaktallergien insbesondere auf Inhaltsstoffe der von Ihnen zum Teil jahrelang angewandten Lokaltherapie haben. Die Lokaltherapeutika sollten daher in ihrer Zusammensetzung so einfach wie möglich sein und die Anzahl der verwendeten Substanzen so gering wie möglich gehalten werden.

In der lokalen Ulkustherapie sind heutzutage 2 Behandlungsmethoden akzeptiert, nämlich eine phasenadaptierte und eine phasenübergreifende Wundbehandlung. Diese Therapiekonzepte beziehen sich auf die 3 Phasen der Wundheilung, die wie folgt ablaufen:
- Exsudationsphase
   (häufig ausgelöst durch eine Superinfektion des Ulkus)
- Granulationsphase
- Epithelisierungsphase

Die phasenadaptierte Wundheilung entspricht der konventionellen, althergebrachten Therapie, muß ständig verändert werden und ist aufwendig, aber preiswert. Die phasenübergreifende Therapie, die sich moderner Wunddressings bedient, mit denen während aller 3 Phasen auf die gleiche Weise behandelt werden kann, ist wesentlich einfacher zu handhaben, aber auch wesentlich teurer. Beide Therapieverfahren sind im therapeutischen Effekt miteinander vergleichbar.

Die phasenadaptierte Wundbehandlung greift in die 3 Wundheilungsphasen wie folgt ein:
- Reinigung
- Granulationsförderung
- Förderung der Epithelisierung durch Austrocknen

Ziel der Reinigung ist die Herstellung eines sauberen Wundgrundes. Dazu müssen zunächst Salbenreste und Krusten beseitigt und eventuell vorhandene Nekrosen abgetragen werden. Letzteres geschieht am besten mit dem scharfen Löffel und mit Pinzette und Schere. Zusätzlich kann man enzymatische Salben verwenden, die vorzugsweise denaturiertes Protein abbauen. Sie enthalten Stoffe wie Trypsin, Chymotrypsin, Kollagenase, Fibrolysin, Streptokinase etc. Die Ulkusumgebung sollte dabei immer durch Auftragen von harter Zinkpaste geschützt werden. Parallel hierzu erfolgt die regelmäßige Desinfektion z.B. mit Kaliumpermanganat - oder Rivanol^{®} -Bädern.

Auf die Anwendung lokaler Antibiotika sollte verzichtet werden, da zum einen die Resistenzentwicklung, zum anderen die Sensibilisierungsrate hoch ist.

Die Granulation wird durch mechanische Reizung angeregt. Deshalb ist heute noch die Curettage mit dem scharfen Löffel ein sehr erfolgreiches Mittel. Etwas milder kann man den Wundgrund mit Seesand, Kristallzucker oder Dextranpuder reizen. Auch hypertone Traubenzucker- und Natriumchloridlösungen sind granulationsfördernd. Die früher üblichen Behandlungen mit Salben, Farbstoffen und Mineralölraffinaden werden heute nicht mehr empfohlen, da sie nicht selten bei längerer Anwendung zu Kontaktallergien führen.

In der Epithelisierungsphase soll das empfindliche neugebildete Gewebe geschützt und die Epithelisierung gefördert werden. Die Epithelisierung setzt ein, wenn das Granulationsgewebe das obere Hautniveau erreicht hat. Als epithelisierungsfördernde Wundauflage eignen sich eine Reihe von Präparate, so z.B. Hydrogele, Hydrokolloide, Schaumstoffkompressen und Gazegitter. Auch kann die Transplantation von Spalthaut vorgenommen werden.

Bei der phasenübergreifenden Wundbehandlung werden z.B. trockene Wundauflagen und Gele (Xerodressing und Xerogel) sowie hydrokolloidale Wundauflagen (Hydrokolloiddressings) verwendet. Sie entfalten in jeder Phase ihre Wirkung und können als Monotherapie zur Behandlung des Ulcus cruris eingesetzt werden. Zu ihren Eigenschaften gehören die Exsudatretention, die Ulkusreinigung sowie die Förderung der Granulation und Epithelisierung.

Nach Publikationen aus England, Dänemark, Kanada, Schweden, Südafrika und den USA hatten 1 bis 11 % aller Patienten in Krankenhäusern Dekubitalulzera: Ursache hierfür sind die steigende Lebenserwartung, die Zunahme gefährdeter Patienten sowie die Tatsache, daß immer mehr schwerstkranke und schwerverletzte Patienten überleben. Hinzu kommen der wachsende Mangel an Pflegekräften und deren ständige Überlastung.

Ein längere Zeit anhaltender Druck auf die Haut mit einer Störung der Mikrozirkulation ist der wichtigste Faktor, der zu einem Dekubitus führt (National Pressure Ulcer Advisory Panel (1989)). Eine wichtige Rolle spielen auch Scherkräfte, die auf die Haut und das darunterliegende Gewebe einwirken, sowie Reibung und Feuchtigkeit. Je unbeweglicher ein Patient ist, desto anhaltender wird die Haut an der gleichen Stelle belastet. In Rückenlage ist dies vor allem die Hautregion über dem Kreuzbein. Besonders gefährdet für Dekubitus sind daher ältere Patienten, die immobil sind, deren Durchblutung gestört ist oder die an neurologischen Störungen leiden. Neben einer Druckentlastung der betroffenen Hautareale erfolgt die Lokalbehandlung von Dekubitalulzera grundsätzlich in der gleichen Weise wie die von Unterschenkelgeschwüren.

Es wurde nun gefunden, daß Puderzubereitungen von Hydroxypyridonderivaten bestens zur einfachen und kostengünstigen phasenübergreifenden Lokaltherapie von Unterschenkel- und Druckgeschwüren geeignet sind. Dieser Befund ist insofern überraschend, als hydroxypyridon-haltige Zubereitungen bislang ausschließlich als antimikrobielle Mittel Verwendung finden.

Die Erfindung betrifft daher die Verwendung von Puderzubereitungen enthaltend mindestens eine Verbindung der Formel I 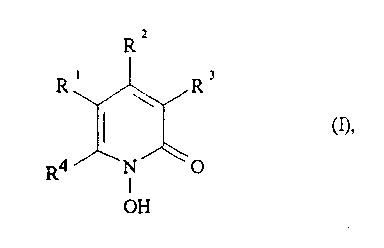 worin R¹, R² und R³, die gleich oder verschieden sind, Wasserstoffatom oder Alkyl mit 1-4 Kohlenstoffatomen bedeuten, und R⁴ einen gesättigten Kohlenwasserstoffrest mit 6 bis 9 Kohlenstoffatomen oder einen Rest der Formel II bedeutet, 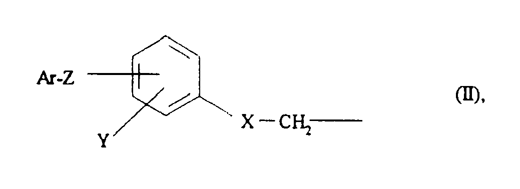 wobei
- X: S oder O bedeutet,
- Y: Wasserstoffatom oder bis zu 2 Halogenatome wie Chlor und/oder Brom bedeutet,
- Z: eine Einfachbindung oder die zweiwertigen Reste O, S, -CR₂-, worin R Wasserstoffatom oder C₁-C₄-Alkyl bedeutet, oder andere zweiwertige Reste mit 2-10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄ Alkylgruppen abgesättigt sind, bedeutet,
- Ar: ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann, bedeutet,
zur Herstellung eines Arzneimittels zur Behandlung von Unterschenkelgeschwüren und/oder Dekubitalulzera.

Der Begriff "gesättigten Kohlenwasserstoffrest" bedeutet, daß der Rest geradkettig, verzweigt oder cyclisch sein kann und keine aliphatischen Mehrfachbindungen enthält. Beispiele für den Rest R⁴ sind Hexyl, Heptyl, Octyl, Cyclohexyl oder Cycloheptyl.

In der Verbindung der Formel I ist die Bedeutung für den Kohlenwasserstoffrest R⁴ bevorzugt ein (C₆-C₈)-Alkyl oder Cyclohexyl, der auch über eine Methylen- oder Ethylengruppe an den Pyridonring gebunden sein oder eine Endomethylgruppe enthalten kann. R⁴ kann auch einen aromatischen Rest darstellen, der jedoch vorzugsweise über wenigstens ein aliphatisches C-Atom an den Pyridonrest gebunden ist.

In den Resten "Z" sind die C-Kettenglieder vorzugsweise CH₂-Gruppen. Wenn die CH₂-Gruppen durch (C₁-C₄)-Alkylgruppen substituiert sind, sind CH₃ und C₂H₅ bevorzugte Substituenten.

Beispielhafte Reste "Z" sind:
- O-, -S-, -CH₂-, -(CH₂)ₘ-(m=2-10), -C(CH₃)₂-, -CH₂O-, OCH₂-, -CH₂S-, -SCH₂-, -SCH(C₂H₅)-, -CH=CH-CH₂O-, -OCH₂CH=CHCH₂O-, -OCH₂CH₂O-, -OCH₂CH₂CH₂O-, -SCH₂CH₂CH₂S-, -SCH₂CH₂CH₂CH₂O-, -SCH₂CH₂OCH₂CH₂O-, -SCH₂CH₂OCH₂CH₂OCH₂CH₂S-, oder
- SCH₂C(CH₃)₂CH₂S-.

Der Rest "S" bedeutet Schwefelatom, der Rest "0" bedeutet Sauerstoffatom. Der Begriff "Ar" bedeutet Phenyl und kondensierte System mit bis zu 2 Ringen wie Naphthyl, Tetrahydronaphthyl und Indenyl, sowie isolierte Systeme mit bis zu 2 Ringen wie solche, die sich vom Biphenyl, Diphenylalkanen, Diphenylethern und Diphenylthioethern ableiten. Der Begriff "Alkyl mit 1 bis 4 Kohlenstoffatomen" steht für Reste wie Methyl, Ethyl, n-Propyl, iso-Propyl, n-Butyl, iso-Butyl oder tertiär-Butyl.

Wichtige Vertreter der durch die Formel I charakterisierten Verbindungsklasse sind:
6-[4-(4-Chlor-phenoxy)-phenoxymethyl]-1-hydroxy-4-methyl-2-pyridon;
6-[4-(2,4-Dichlor-phenoxy)-phenoxymethyl]-1-hydroxy-4-methyl-2-pyridon;
6-(Biphenylyl-4-oxy-methyl)-1-hydroxy-4-methyl-2-pyridon;
6-(4-Benzyl-phenoxymethyl)-1-hydroxy-4-methyl-2-pyridon;
6-[4-(2,4-Dichlorbenzyloxy)-phenoxymethyl]-1-hydroxy-4-methyl-2-pyridon;
6-[4-(4-Chlor-phenoxy)-phenoxymethyl]-1-hydroxy-3,4-dimethyl-2-pyridon;
6-[4-(2,4-Dichlor-benzyl)-phenoxymethyl]-1-hydroxy-3,4-dimethyl-2-pyridon;
6-[4-(Cinnamyloxy)-phenoxymethyl]-1-hydroxy-4-methyl-2-pyridon;
1-Hydroxy-4-methyl-6-[4-(4-trifluormethyl-phenoxy)-phenoxymethyl]-2-pyridon;
1-Hydroxy-4-methyl-6-cyclohexyl-2-pyridon;
1-Hydroxy-4-methyl-6-(2,4,4-trimethylpentyl)-2-pyridon,
1-Hydroxy-4-methyl-6-n-hexyl, -6-iso-hexyl-, 6-n-heptyl- oder -6-iso-heptyl-2-pyridon, 1-Hydroxy-4-methyl-6-octyl- oder -6-iso-octyl-2-pyridon, insbesondere 1-Hydroxy-4-methyl-6-cyclohexylmethyl- oder -6-cyclohexylethyl-2-pyridon, wobei der Cyclohexylrest jeweils auch einen Methylrest tragen kann,
1-Hydroxy-4-methyl-6-(2-bicyclo[2,2,1]heptyl)-2-pyridon, 1-Hydroxy-3,4-dimethyl-6-benzyl- oder -6-dimethylbenzyl-2-pyridon oder 1-Hydroxy-4-methyl-6-(β-phenylethyl-2-pyridon.

Die oben genannten Verbindungen der Formel I können sowohl in freier Form als auch als Salze eingesetzt werden, die Verwendung in freier Form ist bevorzugt.

Die in den Zubereitungen einzusetzenden Wirkstoffe der Verbindung der Formel I können beispielsweise nach Verfahren gemäß US 2 540 218 oder US 4 797 409 hergestellt werden.

Für den erfindungsgemäßen Einsatz der genannten Verbindungen kommen Puderzubereitungen in Betracht.

In den erfindungsgemäßen Zubereitungen wird der Wirkstoff in Mengen eingearbeitet, die üblicherweise zwischen 0,05 und 5 %, vorzugsweise zwischen 0,1 und 1 % liegen.

Mit den erfindungsgemäßen Arzneimitteln läßt sich bei der Lokalbehandlung von Unterschenkelgeschwüren und Dekubitalulzera eine durchgreifende Heilung erzielen.

Als Pudergrundlage kommen native Substanzen wie Saccharose, Lactose, Gelatine, Pektin, Karrageen, Agar, Tragant und Alginate, halbsynthetische Pudergrundlagen wie Celluloseether, Stärke-, Dextran- und Pektinderivate sowie vollsynthetische Substanzen wie Polyvinylpyrrolidone in Frage. Besonders geeignet sind modifizierte Stärketypen. Sie werden in Mengen von 95,0 bis 99,4 Gewichtsteilen auf 100 Gewichtsteile Endprodukt eingesetzt.

Als weiteres Hilfsmittel für die erfindungsgemäße pharmazeutische Zubereitung eignet sich hochdisperses Siliciumdioxid zur Verbesserung der Fließ- und Streufähigkeit.

Die Herstellung der Zubereitungen erfolgt in an sich bekannter Weise durch Mischung der einzelnen Komponenten und einer - soweit erforderlich - der jeweiligen Zubereitung angepaßten Weiterverarbeitung.

Die vorliegende Erfindung wird durch die folgenden Beispiele näher erläutert. Soweit nicht anderes vermerkt, sind die Mengenangaben auf das Gewicht bezogen.

### Beispiel 1

Eine erfindungsgemäße Zubereitung weist folgende Zusammensetzung auf:

| | |
|---|---|
| 6-Cyclohexyl-1-hydroxy-4-methyl-2(1H)-pyridon,2-Aminoethanolsatz | 1,00 g |
| Hochdisperses Siliciumdioxid | 0,01 g |
| Modifizierte Maisstärke, phosphatiert | 98,99 g |

### Beispiel 2

Eine erfindungsgemäße Zubereitung weist folgende Zusammensetzung auf:

| | |
|---|---|
| 1-Hydroxy-4-methyl-6-(2,4,4-trimethylpentyl)-2(1H)pyridon | 0,50 g |
| Saccharose | 99,50 g |

### Beispiel 3

Eine erfindungsgemäße Zubereitung weist folgende Zusammensetzung auf:

| | |
|---|---|
| 6-[4-(4-Chlor-phenoxy)-phenoxymethyl]-1-hydroxy-4-methyl-2(1H)-pyridon | 0,25 g |
| Lactose | 99,75 g |

### Beispiel 4

### Wirksamkeitsprüfung

In einer klinischen Studie an Patienten mit schlecht heilenden Unterschenkelgeschwüren konnte gezeigt werden, daß nach einer Behandlungszeit von 6 Wochen mit der erfindungsgemäßen Zubereitung gemäß Beispiel 1 die Ulkusfläche um 80 % abnahm. Im Vergleich dazu verringerte sich die Größe der Wunde nach Standardtherapie (Wasserstoffperoxid, Kaliumpermanganat, Natriumchloridlösung) nur um 30 %.

## Patentansprüche

1. Die Verwendung von Puderzubereitung enthaltend mindestens ein 1-Hydroxy-2-pyridon der Formel I 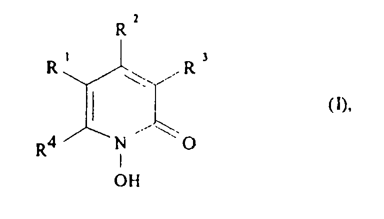 wobei
R¹, R² und R³, die gleich oder verschieden sind, Wasserstoffatom oder Alkyl mit 1-4 Kohlenstoffatomen bedeuten, und
R⁴ einen gesättigten Kohlenwasserstoffrest mit 6 bis 9 Kohlenstoffatomen oder einen Rest der Formel II bedeutet 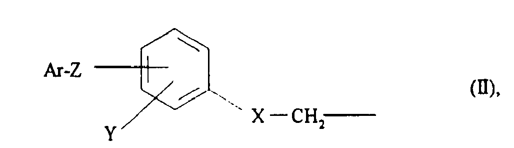 wobei
X S oder O bedeutet,
Y Wasserstoffatom oder bis zu 2 Halogenatome wie Chlor und/oder Brom bedeutet,
Z eine Einfachbindung oder die zweiwertigen Reste O, S, -CR₂-, worin R Wasserstoffatom oder (C₁-C₄)-Alkyl bedeutet, oder andere zweiwertige Reste mit 2-10 kettenförmig verknüpften C- und gegebenenfalls 0- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch Wasserstoffatome und/oder (C₁-C₄)-Alkylgruppen abgesättigt sind, bedeutet,
Ar ein aromatisches Ringsystem mit bis zu zwei Ringen, das durch bis zu drei Reste aus der Gruppe Fluor, Chlor, Brom, Methoxy, (C₁-C₄)-Alkyl, Trifluormethyl und Trifluormethoxy substituiert sein kann, bedeutet,
zur Herstellung eines Arzneimittels zur Behandlung von Unterschenkelgeschwüren und/oder Dekubitalulzera.

2. Verwendung gemäß Anspruch 1, **dadurch gekennzeichnet, daß** man die Verbindung der Formel I einsetzt, worin Ar ein bicyclisches System darstellt, das sich vom Biphenyl, Diphenylalkan oder Diphenylether ableitet.

3. Verwendung gemäß Anspruch 1 oder 2, **dadurch gekennzeichnet, daß** die Verbindung der Formel I in der Position R⁴ einen Cyclohexylrest enthält.

4. Verwendung gemäß einem oder mehreren der Ansprüche 1 bis 3, **dadurch gekennzeichnet, daß** die Verbindung der Formel I in der Position R⁴ einen Octylrest der Formel -CH₂-CH(CH₃)-CH₂-C(CH₃)₃ enthält.

5. Verwendung gemäß Anspruch 1, **dadurch gekennzeichnet, daß** man
1-Hydroxy-4-methyl-6-[4-(4-chlorphenoxy)-phenoxymethyl]-2-(1H)pyridon,
1-Hydroxy-4-methyl-6-cyclohexyl-2-(1-H)-pyridon oder 1-Hydroxy-4-methyl-6-(2,4,4-trimethylpentyl)-2-(1 H)-pyridon einsetzt.

6. Verwendung gemäß einem oder mehreren der Ansprüche 1 bis 5, **dadurch gekennzeichnet, daß** als Pudergrundlage modifizierte Maisstärke, Saccharose, Lactose oder eine Mischung davon eingesetzt wird.

7. Verwendung gemäß einem oder mehreren der Ansprüche 1 bis 6, **dadurch gekennzeichnet, daß** man zusätzlich hochdisperses Siliciumdioxid einsetzt.

8. Verwendung gemäß einem oder mehreren der Ansprüche 1 bis 7, **dadurch gekennzeichnet, daß** man das 1-Hydroxy-2-pyridon der Formel I in einer Menge von 0,05 % bis 5 %, vorzugsweise von 0,1 % bis 1 %, einsetzt.

## Claims

1. Use of powder preparation comprising at least one 1-hydroxy-2-pyridone of the formula I 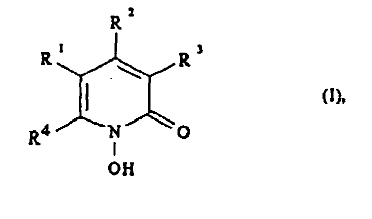 where
R¹, R² and R³, which are identical or different, are hydrogen atom or alkyl with 1-4 carbon atoms, and
R⁴ is a saturated hydrocarbon radical with 6 to 9 carbon atoms or a radical of the formula II 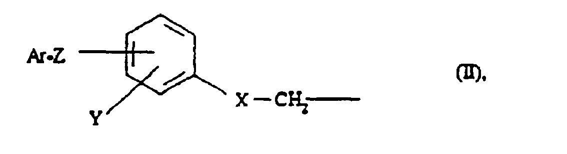 where
X is S or O,
Y is hydrogen atom or up to 2 halogen atoms such as chlorine and/or bromine,
Z is a single bond or the divalent radicals O, S, -CR₂-, in which R is hydrogen atom or (C₁-C₄)-alkyl, or other divalent radicals with 2-10 C and, where appropriate, O and/or S atoms linked in the form of a chain, it being necessary - if the radicals contain 2 or more O and/or S atoms - for the latter to be separated from one another by at least 2 C atoms, and it also being possible for 2 adjacent C atoms to be linked together by a double bond, and the free valencies of the C atoms being saturated by hydrogen atoms and/or (C₁-C₄)-alkyl groups,
Ar is an aromatic ring system which has up to two rings and which may be substituted by up to three radicals from the group of fluorine, chlorine, bromine, methoxy, (C₁-C₄)-alkyl, trifluoromethyl and trifluoromethoxy, for the production of a pharmaceutical for the treatment of leg ulcers and/or decubitus ulcers.

2. Use according to Claim 1, **characterized in that** the compound of the formula I in which Ar is a bicyclic system which is derived from biphenyl, diphenylalkane or diphenyl ether is employed.

3. Use according to Claim 1 or 2, **characterized in that** the compound of the formula I contains a cyclohexyl radical in position R⁴.

4. Use according to one or more of Claims 1 to 3, **characterized in that** the compound of the formula I contains an octyl radical of the formula -CH₂-CH(CH₃)-CH₂-C(CH₃)₃ in position R⁴.

5. Use according to Claim 1, **characterized in that**
1-hydroxy-4-methyl-6-[4-(4-chlorophenoxy)phenoxymethyl]-2-(1H)pyridone, 1-hydroxy-4-methyl-6-cyclohexyl-2-(1-H)-pyridone or 1-hydroxy-4-methyl-6-(2,4,4-trimethylpentyl)-2-(1H)-pyridone is employed.

6. Use according to one or more of Claims 1 to 5, **characterized in that** modified corn starch, sucrose, lactose or a mixture thereof is employed as powder base.

7. Use according to one or more of Claims 1 to 6, **characterized in that** highly disperse silica is additionally employed.

8. Use according to one or more of Claims 1 to 7, **characterized in that** the 1-hydroxy-2-pyridone of the formula I is employed in an amount of 0.05% to 5%, preferably of 0.1% to 1%.

## Revendications

1. Utilisation d'une composition pulvérulente contenant au moins une 1-hydroxy-2-pyridone de formule I 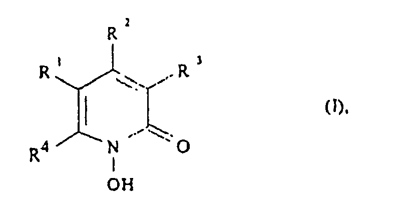 où
R¹, R² et R³, qui sont identiques ou différents, signifient un atome d'hydrogène ou un groupe alkyle comprenant 1-4 atomes de carbone, et
R⁴ signifie un radical hydrocarboné saturé comprenant 6 à 9 atomes de carbone ou un radical de formule II 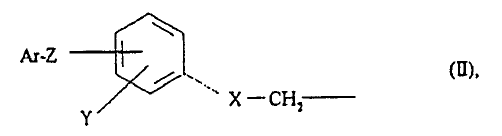 où
X signifie S ou O,
Y signifie un atome d'hydrogène ou jusqu'à 2 atomes d'halogène, tels que le chlore et/ou le brome,
Z signifie une simple liaison ou les radicaux divalents O, S, -CR₂-, où R signifie un atome d'hydrogène ou (C₁-C₄)-alkyle, ou d'autres radicaux divalents comprenant 2-10 atomes de C et le cas échéant O et/ou S liés en forme de chaîne, où - lorsque les radicaux contiennent 2 atomes de O et/ou de S ou plus - ces derniers doivent être séparés l'un de l'autre par au moins deux atomes de carbone et où 2 atomes de carbone adjacents peuvent également être liés l'un à l'autre par une double liaison et les valences libres des atomes de carbone sont saturées par des atomes d'hydrogène et/ou des groupes (C₁-C₄)-alkyle,
Ar signifie un système cyclique aromatique comprenant jusqu'à deux cycles, qui peuvent être substitués par jusqu'à trois radicaux du groupe formé par le fluor, le chlore, le brome, méthoxy, (C₁-C₄)-alkyle, trifluorométhyle et trifluorométhoxy,
pour la préparation d'un médicament destiné au traitement d'ulcères de la partie inférieure de la jambe et/ou des escarres.

2. Utilisation selon la revendication 1, **caractérisée en ce qu'**on utilise le composé de formule I dans laquelle Ar représente un système bicyclique, qui est dérivé d'un biphényle, d'un diphénylalcane ou d'un diphényléther.

3. Utilisation selon la revendication 1 ou 2, **caractérisée en ce que** le composé de formule I contient un radical cyclohexyle en position R⁴.

4. Utilisation selon l'une ou plusieurs des revendications 1 à 3, **caractérisée en ce que** le composé de formule I contient, en position R⁴, un radical octyle de formule -CH₂-CH(CH₃)-CH₂-C(CH₃)₃.

5. Utilisation selon la revendication 1, **caractérisée en ce qu'**on utilise de la 1-hydroxy-4-méthyl-6-[4-(4-chlorophénoxy)-phénoxyméthyl]-2-(1H)pyridone, de la 1-hydroxy-4-méthyl-6-cyclohexyl-2-(1H)-pyridone ou de la 1-hydroxy-4-méthyl-6-(2,4,4-triméthylpentyl)-2-(1H)-pyridone.

6. Utilisation selon l'une ou plusieurs des revendications 1 à 5, **caractérisée en ce qu'**on utilise, comme base de la poudre, de l'amidon de maïs modifié, du saccharose, du lactose ou un mélange de ceux-ci.

7. Utilisation selon l'une ou plusieurs des revendications 1 à 6, **caractérisée en ce qu'**on ajoute en outre du dioxyde de silicium hautement dispersé.

8. Utilisation selon l'une ou plusieurs des revendications 1 à 7, **caractérisée en ce qu'**on utilise de la 1-hydroxy-2-pyridone de formule I en une quantité de 0,05% à 5%, de préférence de 0,1% à 1%.
